# EUROPEAN PATENT APPLICATION

(11) **EP 4 169 498 A1**
(43) Date of publication of application: **26.04.2023**
(21) Application number: 21204531.4
(22) Date of filing: 25.10.2021
(51) Int. Cl.: A61H 99/00, H04S 1/00, A61M 21/00

(54) **METHOD FOR GENERATING STIMULATION-AUDIO-FILES**

(71) Applicant: Bottneuro AG, 4057 Basel (CH)
(72) Inventor: Osmani, Bekim, 4056 Basel (CH)
(74) Representative: Mertzlufft-Paufler, Cornelius

(57) **Abstract**

A method and corresponding technical means are proposed for generating stimulation-audio-files (2) from almost any suitable input-audio-file (1), for example from a digital music stream. The stimulation-audio-file (2) offers two audio signals (3,4), which are each generated from the input-audio-file (1) by applying a respective specific disturbance (5,6) to the input-audio-file (1). As a result, the stimulation-audio-file (2) still delivers the main content of the input-audio-file (1) but its audio signals (3, 4) are also modulated by a respective modulation frequency that is imprinted into the respective audio signal (3,4) by the respective disturbance (5,6). When played-back stereophonically to a patient, the generated stimulation-audio-file (2) can thus efficiently stimulate the auditory cortex of the patient's brain, while the patient is fully or at least for the most part unaware of the stimulation (Fig.3).

## Description

The present disclosure relates to the field of audio-stimulation of the human brain for therapeutical purposes. More precisely, the disclosure concerns a method, which can be computer-implemented, for generating stimulation-audio-files that may be employed (i.e. the generated stimulation-audio-files are usable) for that purpose. The method generates, preferably using a computer, at least one stimulation-audio-file from an, preferably digital, input-audio-file.

As one prominent example of audio-stimulation for therapeutical purposes, it is well known that the progression of Alzheimer's disease can be halted or at least slowed down by audio stimulation of the patient's brain, with most patients responding to frequencies from 20 to 80 Hz. However, if, for example, an audio signal of about 40 Hz is used directly, this is often perceived by the patient as very disturbing, so that the audio stimulation cannot be performed over longer periods of time. For example, it is almost impossible for a patient to endure such a signal through his ears for more than 15 minutes.

In this context, the invention aims at providing the technical means for a more pleasant and convenient way of effective audio-stimulation of the human brain, which does not stress the patient as much as existing methods. Moreover, the technical means shall be easily accessible to a large group of patients, also in less developed countries.

In accordance with the present invention, a method is provided according to claim 1, which solves the afore-mentioned problem. In particular the invention proposes a method for generating audio-stimulation-files as introduced at the beginning, which, in addition, is characterized in that the at least one stimulation-audio-file is appropriate for stereophonic playback by comprising a first audio signal, which can be played to a left ear of a patient and a second audio signal, which can be played to a right ear of the patient, and wherein the first and second audio signal are each modulated by a respective characteristic disturbance.

Surprisingly, it has been found that the patient is essentially unaware of the disturbances imprinted into the two audio signals, provided the disturbances are suitably set up, and can thus enjoy his favorite music song (= input audio file) almost undisturbed. At the same time, however, the auditory nerve converts the modulated audio signals into corresponding electrical signals of the auditory nerve, which is passed on via neurons to the auditory cortex, which is an area in the human brain relevant for human hearing. Via the neurons, the stimulation frequency thus also reaches deeper brain regions in the form of a corresponding nerve signal and can stimulate glial cells in these regions. Since the glial cells essentially float in the brain, the glial cells activated by the generated nerve signals can also move on to other areas of the brain to exert a positive effect (i.e. inhibitory effect) on the progression of Alzheimer's disease, as one example of a therapeutical effect that can be achieved by employing an audio-stimulation-file generated with a method according to the invention.

Obviously, depending on the application, it may be beneficial to employ one digital file for each of the two audio signals generated from the input-audio-file, i.e. the two audio signals do not necessarily need to be squeezed into a common digital file / a single stimulation-audio-file.

The input-audio-file and/or the stimulation-audio-file may be in the form of a digital audio file of a given time length / duration; or, for example, these files may be in the form of a continuous digital data stream, e.g. in case the input-audio-file originates from a digital data stream, in particular from the internet or a broadcasting service. Most suitable for the purpose of convenient audio-stimulation of a patient's auditory cortex in the brain will be an input-audio-file that the patient enjoys listening to.

Moreover, the input-audio-file may already offer two audio signals adapt for stereophonic playback. In this case, each of these two input audio signals may be modulated with the respective disturbance to yield the desired first and second audio-signal. In case, the input-audio-file offers only one single audio signal, the two audio signals may be generated from one audio-signal of the input-audio-file.

In electronics and telecommunications, modulation is understood as the process of varying one or more properties of a periodic waveform, called the carrier signal, with a separate signal called the modulation signal that typically contains information to be transmitted. For example, the modulation signal might be an audio signal or a digital signal representing a sequence of binary digits, for example a bitstream from a computer. The carrier is typically higher in frequency than the modulation signal. The purpose of modulation in electronics and telecommunications is to impress the information on the carrier wave, which is used to carry the information to another location.

In the case of the invention, the original input-audio-file may be considered as the carrier signal and the characteristic disturbance may be considered as the modulation that is impressed into the input-audio-file with the respective modulation frequency, because it is the momentary frequency of the disturbance that will generate corresponding nerve signals in the brain, which are relevant for the targeted therapeutic effect. In other words, when a simulation-audio-file generated with the method is played to a patient's ears, the modulation frequency (e.g. a momentary frequency of the disturbance) will be sensed by the human ear and be transformed by the ear into a nerve signal at the modulation frequency which will then stimulate the auditory cortex of the brain. As a result, the auditory cortex will be stimulated at a stimulation frequency that equals the (momentary) modulation frequency present in the stimulation-audio-file.

The modulation of the respective audio signal by the corresponding disturbance may be implemented by applying a respective transfer-function T(t, f) to the respective audio signal (as an input) to yield the modulated audio signal (as an output). The transfer-function may vary over time t and frequency f, i.e. different frequencies of the input audio signal may be modulated differently and/or a different times.

It is further obvious, that the first and second audio signal can be used as a left and right audio signal to be delivered to the left/right ear of a patient in stereophonic playback.

Most preferably, the respective audio signal may be similar or even identical to the original input-audio-file (in particular to a respective left/right audio signal of the input-audio-file), when the disturbance is not present. In other words, the transfer function may be equal to one for most of the time during a period T, and unequal to one during a certain time duration t of the disturbance.

According to the invention, there exist further advantageous embodiments solving the afore mentioned problem, which are described in the sub-claims and in the following, and whose features may also be combined, if appropriate for the respective application.

For example, one embodiment suggests that at least one or both of the respective disturbances is/are an aperiodic disturbance. That is, the period of time T between two consecutive disturbances by which one of the two generated first and second audio signals is modulated may vary over time.

According to an alternative embodiment, the respective disturbance is a periodic disturbance. In this case, the period of time T between two consecutive disturbances by which one of the two generated first and second audio signals is modulated will be constant over time. In other words, the modulation frequency by which the disturbance modulates the respective audio signal will then be constant. In such an embodiment, the respective disturbance may thus modulate the corresponding audio signal with a respective first and second modulation frequency f1, f2 (respectively, i.e. f1 modulates the first audio signal and f2 modulates the second audio signal).

One possible way of achieving a pleasant play-back for the user/patient is to adjust the method such that only one of the respective disturbances is present in the stimulation audio file at a given point in time. In this case, it is preferred, if at any given point in time during the duration of the stimulation-audio-file, there is always one of the two audio signals audible without any disturbance, in particular even in points in time in which at least one of the two disturbances is present. In other words, always one of the two audio signals may be undisturbed or unmuted at any given point in time.

The presence of the respective disturbance may be understood here as the duration in time, in which the input-audio-file or a (partial) signal from that input-audio-file is altered by the disturbance. The second feature can thus result in a play-back of the generated audio-file, in which - at any given point in time during the play-back - the user will experience an audible signal, either on his left ear or on his right ear.

For example, the disturbance present in the first audio signal and the disturbance present in the second audio signal may show a time shift to each other. In this case, it is preferable if during play-back of the stimulation-audio-file, the disturbances do not overlap in time, i.e. if the disturbances are disjoint in time. The time shift may be constant over time or it may vary over time. However, preferably, the time shift should be large enough, that no overlap between the disturbances occurs.

The mentioned first modulation frequency f1 of the disturbance present in the first audio signal may differ from the second modulation frequency f2 of the disturbance present in the second audio signal. As a result, the the time-lag between the respective disturbances may vary over time. Furthermore, the respective durations T = 1/f, after which a disturbance is repeated, may be different between the first and second audio signal.

According to another embodiment, which also allows a pleasant playback, the durations t1 and t2 of the disturbances and/or a frequency difference between the first and second modulation frequencies f1 and f2 may be chosen such that in more than 80%, preferably in more than 95%, most preferably in more than 99%, of the duration of the stimulation audio-file, there is only one of the respective disturbance present. In other words, in such an embodiment of the method, certain temporal overlaps between the disturbances present in the first and second audio signal are allowed, but they are designed to occur only within a specified fraction of the playback time, for example in less than 20%, preferably less than 5%, or even in less than 1% of the playback time (e.g. both disturbances may be present in less than 1% of the playback time - on average).

The method may also be implemented such that the first modulation frequency f1 of the disturbance present in the first audio signal equals the second modulation frequency f2 of the disturbance present in the second audio signal. In this case, a time-lag between the disturbances can be maintained, such that only one of the disturbances is present at a certain point in time.

Concerning the particular design of the disturbances, which may be described/defined by the mentioned transfer function, in the simplest case, the disturbance present in the first audio signal may equal the disturbance present in the second audio signal, in particular with respect to a signal form or a duration of the disturbance. In other words, a respective transfer function used for generating the first and second audio signals from the input-audio-signal may be of the same type or similar. More precisely, a respective transfer function applied to the input-audio-file to modulate the respective audio signal may be similar, but it may be applied to the input-audio-file with/without a time-shift to produce two different audio signals from the same input-audio-file.

According to a preferred embodiment, the respective modulation frequency f1, f2 (which may be a temporary frequency used for the modulation at a certain point of time or a constant frequency) may lie within a frequency band from 20 Hz to 80 Hz. Alternatively or additionally, the respective modulation frequency f1, f2 may lie within a frequency band of 5 Hz +/- 3 Hz. While the first frequency band is appropriate to induce Gamma-waves in the auditory cortex of the brain, the second frequency band can be used to induce Alpha-Waves in the auditory cortex.

The respective disturbance may be realized as a transient/temporary attenuation, in particular muting, or as a transient/temporary amplification of the respective audio signal. As an example, a possible muting may be achieved by attenuating the amplitude of the audios signal by -30dB over all audio frequencies present in the signal.

According to a more specific embodiment, the respective disturbance may be realized as a transient/temporary attenuation or amplification of a portion of frequencies currently present in the respective audio signal. In such a case, it is preferable, if a frequency band that is attenuated by the respective disturbance lies within 1 to 10 kHz, preferably within 2.5 to 5.5 kHz.

One possible way of generating such frequency specific attenuation or amplification is to apply a highpass and/or a lowpass frequency filter (e.g. by digital signal processing) to the input-audio-file to filter out a certain frequency spectrum from the input-audio-file and to apply the transfer function to the filtered frequency spectrum afterwards. This may be done on multiple filtered spectra, while other spectral portions of the input-audio-file may not be attenuated or amplified, and these spectra may then be reunited to form the respective audio signal to be delivered to the patient.

In preferred embodiments, a duration ti of the respective disturbance may amount to less than 20%, preferably less than 5%, most preferably to less than 1%, of the period Ti= 1/fi of the respective modulation. In other words, a respective duty cycle t/T of the respective disturbance may amount to less than 20%, preferably less than 5%, most preferably to less than 1%. As an example, when using a modulation frequency of f = 40 Hz, corresponding to a period T = 1/f of 25 ms, a disturbance by muting the respective audio signal for t = 5 ms would result in a duty cycle of the disturbance of t/T = 5ms/25ms = 20%. During the rest of the duty cycle, the respective audio signal may be similar or even identical to the original input-audio-file (in particular to a respective left/right audio signal of the input-audio-file).

In particular, the duration ti of the disturbance present in the first audio signal may equal the duration t₂ of the disturbance present in the second audio signal.

According to another preferred embodiment, a fading function (sometimes called windowing function) may be applied to the respective disturbance. This may be done preferably such that the respective disturbance is gradually imposed on the respective audio signal and, most preferably, also gradually remitted from the respective audio signal. In such a case, the full rise and/or decline of the transfer function may each last for at least 10%, preferably for at least 20%, of the duration ti of the respective disturbance, respectively. The fading or windowing function, which may be a sawtooth-, a Hamming-window function or any other suitable function, can thus result in a gradual dying out/decaying of the disturbance (at the end) and/or in a gradual rise/increase of the disturbance (at the beginning). As a result of the fading / windowing, an amplitude of the disturbance may thus vary over time during the duration of the disturbance. The duration of the disturbance may be measured for example by applying a full-width-half-maximum (FWHM) approach to the function describing the fading / windowing.

The overall transfer function mentioned earlier, which may be applied to the input-audio-file to compute the respective audios signal, may thus be designed as the product of a disturbance function (in the simplest case a square or notch function) and a fading/windowing function. The latter may smoothen sharp corners of the disturbance function. This concept is known form digital signal processing, in particular when scanning analogue signals. Here it is applied to achieve a more pleasant (and hence endurable) play-back of a stimulation-audio-file.

According to one particular implementation of the method, at least one of the modulation frequencies f1, f2 may be swept within a modulation frequency band during the duration of the stimulation-audio-file. In this case, the respective modulation frequency f1(t), f2(t) will vary over time, i.e. the due to the frequency sweep, the swept modulation frequency varies over time. The frequency band in which the respective frequency is sweeped can preferably cover a range from 1 to 10 kHz. This approach has the benefit that many possibly effective stimulation frequencies are delivered to the patient.

Another approach is to choose the modulation frequencies, by which the audio signals (3,4) are modulated, based on a patient-specific pre-diagnostic analysis of a particular patient. In other words, the modulation frequencies may be chosen such that an efficient stimulation of the auditory cortex can be expected for a particular patient, in particular based on experimental pre-tests. Preferably, such pre-tests may be performed using an Electroencephalography (EEG) with that patient to reveal to which audio frequencies the patient responds strongly. Moreover, the choice of modulation frequency may be based on a digital bio marker obtained from the patient's response to different modulation frequencies. This approach is beneficial because the frequency of Gamma waves of the modulation to which a patient responds may vary from patient to patient. Electroencephalography (EEG) is an electrophysiological monitoring method to record electrical activity on the scalp that has been shown to represent the macroscopic activity of the surface layer of the brain underneath. It is typically non-invasive, with the electrodes placed along the scalp and can thus be performed with many patients.

According to a highly preferred embodiment, the respective modulation frequencies are within 40 +/-10 Hz. This frequency range has been found to allow efficient audio-stimulation for a large range of patients.

For solving the afore-mentioned problem, a particular use of of head phones is suggested, preferably a use of in-ear headphones, namely for stereophonic replay of a stimulation-audio-file that has been generated with a method according to one of the claims directed towards such a method or as described herein. In such an application case, the stimulation-audio-file may be wirelessly transmitted to the head phones and/or the stimulation-audio-file may be generated (in particular "on the fly" / in real-time) by a mobile electronic device, for example by a smart phone or a tablet.

For solving the afore-mentioned problem, a data processing apparatus is also proposed. This apparatus may be a mobile electronic device, for example. To solve the problem, the apparatus comprises means, which are configured to carry out a method according to one of the according to one of the claims directed towards a method or as described herein.

In particular, the apparatus may comprise a receiver configured to receive the input-audio-file and/or a micro-processor configured to generate the stimulation-audio-file from the input-audio-file and/or an output device configured to output the generated stimulation-audio-file. Most preferably in such a case, the micro-processor may be configured to generate the stimulation-audio-file in real-time, in particular in a continuous play-back mode. This allows use of a continuous live digital data stream as the input-audio-file.

Another solution for benefiting from the method as proposed herein are head phones, preferably wireless and/or in-ear headphones, with an integrated data processing apparatus that is designed as described above. In such a case, it is most convenient for the user, if the data processing apparatus features a wireless receiver for wirelessly receiving the input audio file.

Finally, for solving the problem introduced at the beginning, a computer program is provided, which may in particular be designed as an app to be run on a mobile electronic device. This computer program comprises instructions, which when the program is executed by a computer / by the mobile electronic device, cause the computer / the device to carry out a method according to one of the claims directed towards a method or a described herein.

It is understood that such a program may be provided by means of a computer-readable data carrier comprising instructions, which, when executed by a computer, cause the computer to carry out a method according to one of the claims directed towards a method or a described herein.

Examples of the present invention shall now be described in more detail. With reference to the accompanying drawings, where features with corresponding technical function are referenced with same numerals even when these features differ in shape or design:
- Fig. 1: illustrates the approach of the invention for generating a stimulation-audio-file from a digital input file,
- Fig. 2: illustrates a possible application case, in which a patient is therapeutically treated by playing a stimulation-audio-file, generated with a method according to the invention, to his ears using a pair of wireless in-ear-headphones,
- Fig. 3: illustrates another example of generating a stimulation-audio-file according to the invention, and
- Fig. 4: illustrates the use of a windowing function.

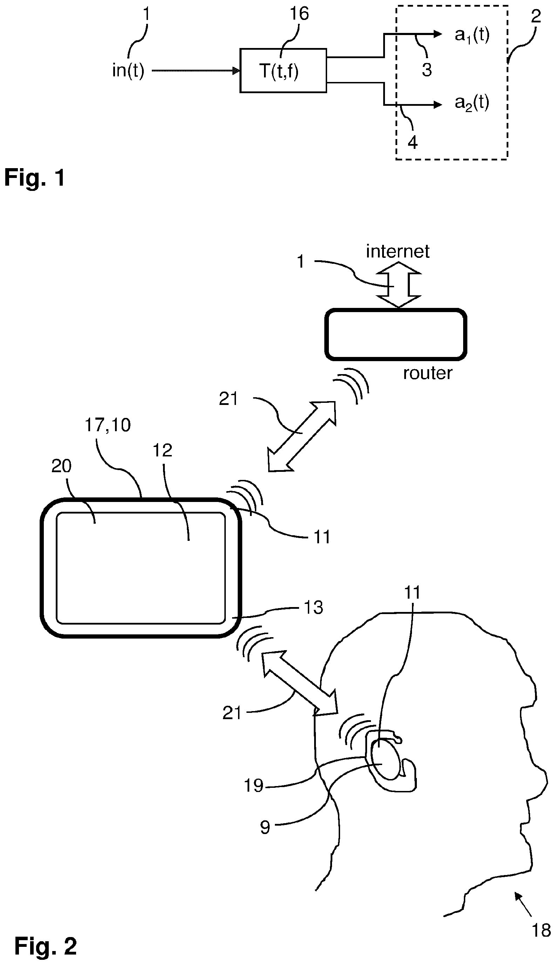

Figure 1 shows a schematic signal flow diagram for illustrating the fundamental approach of the invention. The method according to the invention may use almost any pre-existing digital audio file, e.g. an mp3-file, or a digital live-music-stream from the internet, as an input-audio-file 1 and convert it, in particular in real-time, into a stimulation-audio-file 2 that is adapt for audio-stimulation of the auditory cortex of the brain.

For this purpose, using a suitable computer infra-structure, for example a mobile processor of a smartphone in combination with an app for controlling the computing power of the smartphone, a transfer-function 16 *T(t,f)* is applied to the input-audio-file 1 (denoted as *in(t)* in Figure 1) to generate two separate audio signals 3, 4 (denoted as *a₁(t)* and *a₂(t)* in Figure 1), which can be played-back to a patient. If the two signals 3,4, which are contained in the stimulation-audio-file 2, are played-back simultaneously to the left and right ear of a patient, respectively, a therapeutic effect can be achieved through efficient audio-stimulation of the brain.

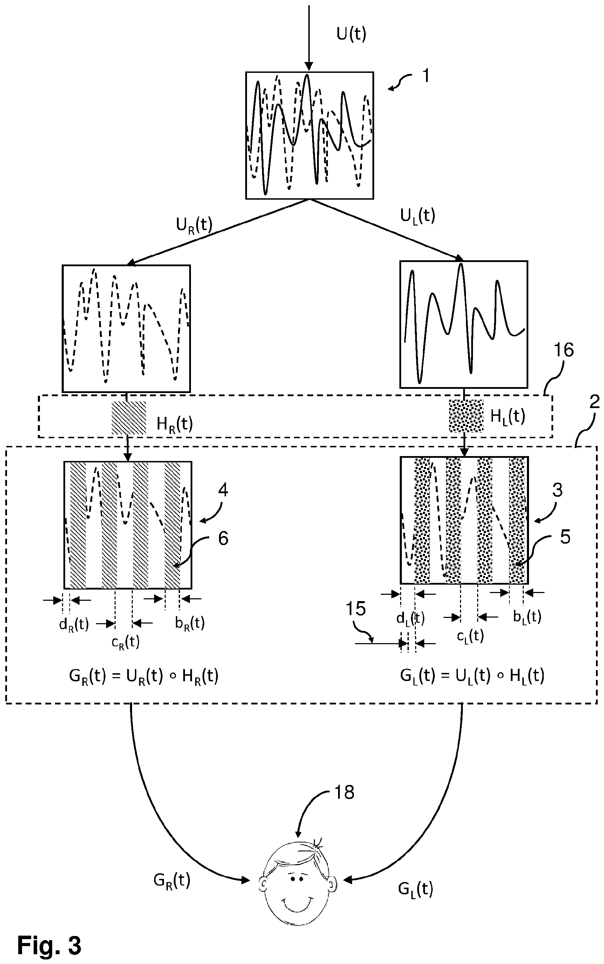

Figure 3 shows another example of a possible implementation of a method according to the invention. In this case, U(t) is the pre-existing input-audio-file 1, which already contains two audio signals, denoted as U_{R}(t) and U_{L}(t), which are adapt for stereophonic playback. The generated stimulation-audio-file 2 also contains two different audio signals, namely a first audio signal 3 (denoted as G_{L}(t) in Figure 3) that can be played to a left ear of the patient 18, and a second audio signal 4 (denoted as G_{R}(t) in Figure 3) that can be played to the patient's right ear.

As indicated in Figure 3, each of the two audio signals 3,4 of the generated stimulation-audio-file 2 are modulated by a respective characteristic disturbance 5, 6. This disturbance may be a jitter, a variation of the amplitude or of the audio signal itself, or any other suitable variation of the respective audio signal 3, 4 that can be sensed by a human ear. This is because the purpose of modulating the respective audio signal 3, 4 with a disturbance 5, 6 is to stimulate the respective auditory nerve (in the left or right ear, respectively) to produce a respective nerve signal whose frequency corresponds to a momentary frequency fi, f₂ of the respective disturbance 5, 6.

As the time-snap-shots of both audio signals 3, 4 in Figure 3 show, the respective disturbance 5/6 is repeated in time. Locally, that is within a certain time span, the frequency of modulation fᵢ thus corresponds to the (local/temporary) repetition rate of the respective disturbance 5/6 in the respective signal 3/4.

The modulation present in the respective audio signals 3, 4 of the stimulation-audio-file 2 is generated by applying a respective individual transfer function 16 (denoted as H_{L}(t) and H_{R}(t) in Figure 3) to the input-audio-file 1, more precisely to each of its signals U_{R}(t) and U_{L}(t). It is obvious, that such an approach allows to imprint different disturbances 5, 6 into the respective audio signal 3, 4 of the stimulation-audio-file 2 by using different individual transfer functions 16 H_{L}(t) and H_{R}(t). The transfer functions can also be varied over time.

As outlined in the claims, a large variety of parameters of the respective disturbance may be varied by adjusting (in particular in real-time) the respective transfer function 16. For example, while the example of Figure 3 shows two periodic disturbances 5, 6, which are each repeated after a fixed time span of Tᵢ, the disturbance can also be applied as an aperiodic disturbance, that is repeated after varying time spans. However, also in the latter case, the disturbance should be designed such that it will generate a specific stimulation frequency or frequency band in the auditory nerve to achieve the desired effect.

The part of the audio signal 3/4 that is distorted by the disturbance 5/6 may be termed a "break" or modified region, while the rest of the audio signal 3/4 may be considered as the "chunk" or non-modified-region. As can be imagined from Figure 3, the duration of chunk versus break in relation to the total time period T after which the disturbance 5/6 is repeated may vary, which can be described as different duty cycles t/T with t representing the duration of the individual disturbance 5/6, which are denoted as *b_{R}(t)* and *b_{L}(t)* in Figure 3, respectively.

Also visible in Figure 3 is the initial delay or offset, i.e. the initial time span after which the disturbance 5/6 sets in. This parameter is denoted as *dL(t)* and *dL(t)* in Figure 3 and can also vary between the two audio signals 3 and 4. As a result, the disturbance 5 present in the first audio signal 3 and the disturbance 6 present in the second audio signal 4 obviously show a time shift 15.

As a further result, in particular when both modulation frequencies of the respective disturbance 5/6 are (at least temporarily) equal, there may exist a time-slot between a disturbance 5 in the first (left) audio signal 3 and a disturbance 6 in the second (right) audio signal 4, in which no disturbance is present at all.

In case the individual durations *b_{R}(t)* and *b_{L}(t)* of the disturbances 5, 6 are chosen appropriately, it may be achieved that at any given point in time during the duration of the stimulation-audio-file 2, there will be always one of the two audio signals 3, 4 audible without any disturbance, even in points in time in which at least one of the two disturbances 5,6 is present.

By such an approach, the patient 18 will remain largely unaware of the individual disturbances and can thus enjoy listening to the song or music represented by the original input-audio-file 1. At the same time, however, a stimulation of the auditory nerve will still be achieved. As a result, pro-longed efficient audio-stimulation becomes feasible, without fatigue of the patient.

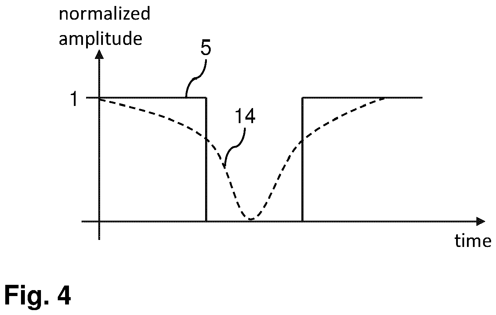

In the example of Figure 3, the disturbances 5 and 6 still show a slight overlap in time, however. While this may be acceptable (i.e. bearable for the patient) in case the disturbance is fading out at its end and slowly rising at its beginning (as will be explained w.r.t. Figure 4), when using a jitter or other more abrupt disturbance, it may be helpful for achieving a pleasant stimulation if the disturbances 5, 6 are disjoint in time such that they do not show any time overlap.

Figure 4 illustrates a disturbance function 5 that is a notch function and would result in an abrupt and total muting of the respective audio signal to which it is applied. Such a disturbance 5 is highly likely to be noticed by the patient 18. As outlined in the claims, a fading or windowing function 14 may therefore be applied to the disturbance 5 such that the respective disturbance 5 is gradually imposed on the audio signal 3 and gradually remitted from the audio signal 3, as visible in Figure 4. Such a disturbance will still deliver the desired audio-stimulation, but will be much less noticed by the patient.

Finally, Figure 2 illustrates a typical application case in which a stimulation-audio-file 2 is generated live / on-the-fly by a tablet 17 that receives a music stream from the internet as an input-audio-file 1 via a wireless data connection 21 that is present between a receiver 11 of the tablet 17 and a router. The tablet 17 offers a micro-processor 12 for computing the digital stimulation-audio-file 2 and a user interface 20 with which the patient 18 can choose the song/stream to be used as the input-audio-file 1.

Via the user interface 20, it is also possible to adjust parameters of the disturbances 5, 6 which are applied by the tablet 17 to the input-audio-file 1 to generate the desired stimulation-audio-file 2. For this purpose, the tablet 17 employs an app which contains a software code that implements a method according to the invention using the computing power of the tablet 17.

The tablet transmits the generated stimulation-audio-file 2 via a second wireless data connection 21 to a set of in-ear head phones 9 (more precisely to a receiver 11 of the head phone 9) that the patient 18 is wearing on his ears 19. By listening to the live-generated stimulation-audio-file 2, the patient 18 can relax and at the same time, an efficient audio-stimulation of the auditory cortex can be achieved.

In summary, a method and corresponding technical means are proposed for generating stimulation-audio-files 2 from almost any suitable input-audio-file 1, for example from a digital music stream. The stimulation-audio-file 2 offers two audio signals 3,4, which are each generated from the input-audio-file 1 by applying a respective specific disturbance 5, 6 to the input-audio-file 1 (modulation of the file with the respective disturbance). As a result, the stimulation-audio-file 2 still delivers the main content of the input-audio-file 1 but its two audio signals 3, 4 are also modulated by a respective modulation frequency that is imprinted into the respective audio signal 3,4 by the respective disturbance 5, 6. When played-back stereophonically to a patient, the generated stimulation-audio-file 2 can thus efficiently stimulate the auditory cortex of the patient's brain, while the patient is fully or at least for the most part unaware of the stimulation.

### List of reference numerals

- 1: input-audio-file
- 2: stimulation-audio-file
- 3: first audio signal
- 4: second audio signal
- 5: disturbance (present in 3)
- 6: disturbance (present in 4)
- 7: first modulation frequency (of 5)
- 8: second modulation frequency (of 6)
- 9: head phones (in particular in-ear-phones)
- 10: data processing apparatus
- 11: receiver
- 12: micro-processor
- 13: output device
- 14: fading function (applied to 5/6)
- 15: time shift (between 5 and 6)
- 16: transfer function (for computing 3/4 from 1)
- 17: mobile electronic device
- 18: patient
- 19: ear (of 18)
- 20: user interface
- 21: data connection (in particular wireless connection)

## Claims

1. **Method,** in particular computer-implemented method, **for generating stimulation-audio-files (2),**
- which may be used for audio-stimulation of a human brain for therapeutic purposes,
- wherein at least one stimulation-audio-file (2) is generated, preferably by a computer, from an input-audio-file (1), **characterized in that,**
- the at least one stimulation-audio-file (2) is appropriate for stereophonic playback by comprising
- a first audio signal (3), which can be played to a left ear of a patient and
- a second audio signal (4), which can be played to a right ear of the patient, and
- wherein the first and second audio signal (3,4) are each modulated by a respective characteristic disturbance (5,6) .

2. Method according to claim 1,
- wherein at least one or both of the disturbances (5,6) is/are an aperiodic disturbance;
or
- wherein the respective disturbance (5,6) is a periodic disturbance,
- in particular wherein the respective disturbance (5,6) modulates the corresponding audio signal (3,4) with a respective first and second modulation frequency fi, f₂ (7,8) .

3. Method according to claim 1 or 2, wherein only one of the respective disturbances (5, 6) is present in the stimulation audio file (2) at a given point in time,
- preferably such that at any given point in time during the duration of the stimulation-audio-file (2) there is always one of the two audio signals (3, 4) audible without any disturbance.

4. Method according to one of the preceding claims, wherein the disturbance (5) present in the first audio signal (3) and the disturbance (6) present in the second audio signal (4) show a time shift (15) to each other,
- preferably such that during play-back of the stimulation-audio-file, the disturbances (5,6) do not overlap in time / are disjoint in time.

5. Method according to one of the claims 1 to 3, wherein the first modulation frequency f₁ (7) of the disturbance (5) present in the first audio signal (3) differs from the second modulation frequency f₂ (8) of the disturbance (6) present in the second audio signal (4),
- in particular such that the time-lag between the respective disturbances (5, 6) varies over time.

6. Method according to one of the claims 1 to 3 or 5, wherein
- the durations t₁ and t₂ of the disturbances (5,6) and/or
- a frequency difference between the first and second modulation frequencies f₁ and f₂ (7,8)
are chosen such that in more than 80%, preferably in more than 95%, most preferably in more than 99% of the duration of the stimulation audio-file (2), there is only one of the respective disturbance (5, 6) present.

7. Method according to one of the preceding claims, wherein the first modulation frequency f₁ (7) of the disturbance (5) present in the first audio signal (3) equals the second modulation frequency f₂ (8) of the disturbance (6) present in the second audio signal (4).

8. Method according to one of the preceding claims, wherein the disturbance (5) present in the first audio signal (3) equals the disturbance (6) present in the second audio signal (4), in particular with respect to a signal form or a duration of the disturbance.

9. Method according to one of the preceding claims, wherein the respective modulation frequency fi, f₂ (7,8) lies
- within a frequency band from 20 Hz to 80 Hz and/or
- within a frequency band of 5 Hz +/- 3 Hz.

10. Method according to one of the preceding claims, wherein the respective disturbance (5,6) is realized
- as a transient/temporary attenuation, in particular muting, or
- as a transient/temporary amplification of the respective audio signal (3,4).

11. Method according to one of the preceding claims, wherein the respective disturbance (5,6) is realized as a transient/temporary attenuation or amplification of a portion of frequencies currently present in the respective audio signal (3,4),
- preferably wherein a frequency band that is attenuated by the respective disturbance (5, 6) lies within 1 to 10 kHz, preferably within 2.5 to 5.5 kHz.

12. Method according to one of the preceding claims, wherein a duration ti of the respective disturbance (5,6) amounts to less than 20%, preferably less than 5%, most preferably to less than 1%, of the period Tᵢ= 1/fᵢ of the respective modulation, and/or
- wherein a respective duty cycle t/T of the respective disturbance (5,6) amounts to less than 20%, preferably less than 5%, most preferably to less than 1%,
- in particular wherein the duration ti of the disturbance (5) present in the first audio signal (3) equals the duration t₂ of the disturbance (6) present in the second audio signal (4).

13. Method according to one of the preceding claims, wherein a fading or windowing function (14), is applied to the respective disturbance (5, 6),
- preferably such that the respective disturbance (5,6) is gradually imposed on the respective audio signal (3,4),
- most preferably and gradually remitted from the respective audio signal (3,4),
- in particular wherein the full rise and/or decline of the transfer function each last for at least 10%, preferably for at least 20% of the duration ti of the respective disturbance (5,6), respectively.

14. Method according to one of the preceding claims, wherein at least one of the modulation frequencies fi, f₂ (7,8) is swept within a modulation frequency band during the duration of the stimulation-audio-file,
- in particular such that due to this frequency sweep the swept modulation frequency varies over time,
- preferably wherein the frequency band covered by the frequency sweep ranges from 1 to 10 kHz.

15. Method according to one of the preceding claims, wherein the modulation frequencies, by which the audio signals (3,4) are modulated, are chosen
- based on a patient-specific pre-diagnostic analysis of a particular patient and/or
- such that an efficient stimulation of the auditory cortex can be expected for a particular patient, in particular
- based on experimental pre-tests, preferably using an Electroencephalography (EEG), with that patient that reveal to which audio frequencies the patient responds strongly and/or
- based on a digital bio marker obtained from the patient's response to different modulation frequencies.

16. Method according to one of the preceding claims, wherein the respective modulation frequencies are within 40 +/-10 Hz.

17. **Use of head phones (9),** preferably use of in-ear headphones, **for stereophonic replay of a stimulation-audio-file** generated with a method according to one of the preceding claims,
- preferably wherein the stimulation-audio-file is
- wirelessly transmitted to the head phones (9) and/or
- generated by a mobile electronic device (17), in particular a smart phone or a tablet.

18. A **data processing apparatus (10),** in particular a mobile electronic device (17), comprising means for and/or configured for carrying out a method according to one of the claims 1 to 16,
- preferably wherein the apparatus (10) comprises
- a receiver (11) configured to receive the input-audio-file (1) and/or
- a micro-processor (12) configured to generate the stimulation-audio-file (2) and/or
- an output device (13) configured to output the generated stimulation-audio-file (2),
- most preferably wherein the micro-processor (12) is configured to generate the stimulation-audio-file (2) in real-time, in particular in a continuous play-back mode.

19. **Head phones (9),** preferably wireless and/or in-ear headphones (9), **with an integrated data processing apparatus (10)** according to the previous claim,
- preferably wherein the data processing apparatus (10) features a wireless receiver (11) for wirelessly receiving the input audio file (1).

20. **Computer program,** in particular designed as an app to be run on a mobile electronic device (17), comprising instructions, which when the program is executed by a computer / the mobile electronic device (17), cause the computer / the device (17) to carry out a method according to one of the claims 1 to 16.

21. **Computer-readable data carrier** comprising instructions, which, when executed by a computer, cause the computer to carry out a method according to one of the claims 1 to 16.
